Europäisches Patentamt

European Patent Office

Office européen des brevets

(19)

(11) Publication number: **0 331 077**
**A2**

# (12) EUROPEAN PATENT APPLICATION

(21) Application number: 89103401.9

(51) Int. Cl.⁴: **G01N 21/76 , G01N 33/58**

(22) Date of filing: 27.02.89

(30) Priority: 27.02.88 JP 44989/88

(43) Date of publication of application:
. 06.09.89 Bulletin 89/36

(84) Designated Contracting States:
AT BE CH DE ES FR GB GR IT LI LU NL SE

(71) Applicant: DOJINDO LABORATORIES
2861 Kengunmachi
Kumamoto-shi Kumamoto-ken(JP)

(72) Inventor: Ueno, Keihei
25-39 Suizenjikoen
Kunmamoto-shi,Kumamoto-ken(JP)
Inventor: Sagara, Fumio
3-1343-7 Ikeda,
Kumamato-shi kumamato-ken(JP)
Inventor: Shiga, Masanobu
1226-39 Oyamacho,Kumamoto-shi
Kumamoto-ken(JP)

(74) Representative: Wey, Hans-Heinrich, Dipl.-Ing.
Patentanwälte Wey & Partner
Widenmayerstrasse 49
D-8000 München 22(DE)

(54) Method of detecting chemiluminescence by photography.

(57) A method of microanalysis, which comprises labeling a substrate containing material to be detected with a chemiluminescent substance, inducing a chemiluminescent reaction at the site of the labeled substrate, and photographically recording the reaction, whereby the site of the chemiluminescent reaction on the labeled substrate is detected and illustrated in a two-dimensional perspective.

EP 0 331 077 A2

# METHOD OF DETECTING CHEMILUMINESCENCE BY PHOTOGRAPHY

The present invention relates to a novel method of microanalysis wherein information regarding the material to be detected is recorded in a two dimensional perspective through the use of chemiluminescence.

Study of the gene at the molecular level has noticeably progressed with the discovery of restriction enzymes, with the development of various kinds of vectors, and with advances in cell cultivation techniques. Techniques for detecting nucleic acid fragments having particular base sequences with probes (for example, the colony hybridization method, the Southern method or Northern method) are indispensable for advancing the study of the gene. The identification of proteins (such as receptors on the surface of cell membranes), as well as oligo-saccharides, has also advanced through the use of the monoclonal antibody method, the ELISA method, and the immunoassay method.

In the case of the Southern autoradiography method, DNA fragments are developed and separated in accordance with the molecular weights thereof on a gel plate by electrophoresis. The fragments are then transferred onto a nitrocellulose filter, and the DNA fragment is hybridized with a radioisotope-labeled DNA probe which is complementary to the base sequence of the intended DNA fragment. Finally, the DNA fragment is detected by autoradiography using X-ray film, confirming the presence or absence of the labeled compound, as well as providing information on the position of the compound on the gel plate. Thus, detection and identification of the intended DNA fragment is possible.

Notwithstanding its high sensitivity, the conventional technique of using radioisotopes for detecting micro substances is extremely disadvantageous. Radioisotopes are unstable, and this severely limits their handling. In addition, since the exposure of X-ray films generally requires from one to several days, results of techniques utilizing such films are not immediately available. An enzyme-labeling method is known, but it is not fully satisfactory since it, too, lacks rapid detectability because of the enzyme reaction.

Recently, luminescence immunoassay has been used as a method of microanalysis. (I. Weeks et al, Clin Sci, 70, 403, 1986, etc.) It has been reported that the sensitivity of the luminescence immunoassay method is not inferior to that of the radio-immunoassay method using $^{125}I$ or $^{3}H$. (J. De. Boever, et al, Anal. Chim. Acta., 170, 117, 1985). However, in the conventional chemiluminescence immunoassay, the detection of chemiluminescence of a chemiluminescent-labeled substance is carried out by actinometry with a photomultiplier. While this method is capable of detecting the presence of a labeled compound, information on the position of the labeled compound, as developed on a two-dimensional plane, cannot be obtained.

A system for detecting DNA by using chemiluminescence has also has been reported, which uses a DNA probe labeled with biotin (L.J. Kricka et al, Anal. Chem., 151, 205, 1985). However, this particular method is limited to dot-hybridization and is therefore not entirely satisfactory.

The present invention provides a faster and more efficient means of detecting various substances such as DNA fragments and proteins than heretofore available. More specifically, the present invention relates to a method of microanalysis which comprises labeling a substrate containing material to be detected with a chemiluminescent substance, inducing a chemiluminescent reaction at the site of the labeled substrate, and photographically recording the reaction, whereby the site of the chemiluminescent reaction on the labeled substrate is detected and illustrated in a two-dimensional perspective.

According to one embodiment of the present invention, the substrate is labeled with an o-phenanthroline derivative, a luminol derivative, an acridinium derivative, an oxalate derivative, a metal ion, a metal ion chelate, or an enzyme.

According to a further embodiment of the present invention, the chemiluminescent substance is contacted with hydrogen peroxide, optionally in the presence of a suitable catalyst, to induce the chemiluminescent reaction. Preferred catalysts are copper (II) ion, osmium (VIII) ion, and iron (III) ion.

According to one embodiment of the present invention, the chemiluminescent reaction is induced in close proximity to highly sensitive instant photographic film. In another embodiment of the present invention, the chemiluminescent reaction is induced in close proximity to a highly sensitive X-ray film. In yet a further embodiment of the present invention, the chemiluminescent reaction is photographed with a camera having highly sensitive film.

A further embodiment of the present invention comprises labeling a substrate with a chemiluminescent substance, placing the labeled substrate on a suitable developing medium, placing the developing medium with the labeled substrate thereon in close proximity to highly sensitive instant photographic film or highly sensitive X-ray film, inducing a chemiluminescent reaction at the labeled site on the substrate, and exposing the film.

One method according to the present invention of labeling the substrate containing the material to be detected comprises introducing a functional group such as an amino group or a carboxyl group into the chemiluminescent substance, optionally in the presence of a catalyst, and reacting the material to be detected such as a nucleic acid or a protein with the chemiluminescent substance via the functional group. The following chemiluminescent reactions are preferred for inducing chemiluminescence in accordance with the present invention.

(1) Reacting an o-phenanthroline derivative with hydrogen peroxide in the presence of a copper (II) or osmium (VIII) catalyst.

(2) Reacting a luminol derivative with hydrogen peroxide in the presence of an iron (III) catalyst.

(3) Reacting an acridinium derivative with hydrogen peroxide.

In processes (1) and (2), the reaction proceeds because of the catalytic action of the metal ion which is present in a trace amount. Accordingly, the substrate containing material to be detected is labeled with a phenanthroline or luminol derivative, or, alternatively, it may be labeled with the metal ion or a chelate thereof. Process (3), on the other hand, is directed to a catalyst-free reaction, and the material to be detected is labeled with an acridinium derivative.

According to one embodiment of the present invention, the chemiluminescent reaction is photographed, for example, with a highly sensitive instant film such as Polaroid Type 612 Film (ASA 20,000) or a high sensitive X-ray film. The film is placed next to a gel plate or nitrocellulose filter developing medium which contains the chemiluminescent-labeled or catalyst-labeled substrate, the film and gel plate or filter being separated by a thin plastic or glass sheet or container. A chemiluminescent reacton is then induced, and the reaction leaves a corresponding two dimensional picture on the exposed film, which may then be developed. Alternatively, the labeled substrate on the gel plate or filter developing medium is made illuminated in the dark room as described above, and the illuminated substrate is directly photographed with a camera having a high sensitive film therein, whereby the same result may be obtained.

One means of labelling and photographing a substrate containing material to be detected (the "intended compound") is to first react 6-N-(4-aminobutyl)-ethyl-2,3-dihydro-1,4-phthalhydrazinedione (hereinafter referred to ABEI) as a luminol derivative with the amino group of the nucleic acid base of an oligonuclectide having a specific base sequence via glutaraldehyde to thereby luminol-label the nucleic acid to give a probe. This labeled nucleic acid is then hybridized on a nitrocellulose filter with a fragment having a complementary sequence to the probe which has been prepared in accordance with a conventional method (T. Maniatis et al, Molecular Cloning A Laboratory Manual, Cold Spring Harbor Laboratory, 1982). The nitrocellulose filter is then tightly attached to a Polaroid instant film via a thin plastic sheet. Next, the shutter cover of the instant film casette is opened in a dark room, and an aqueous mixture solution of hydrogen peroxide and iron (III)-tetraphenylporphyrin complex is added to the nitrocellulose filter to cause chemiluminescence therein. After being exposed for a suitable time, for example about 10 minutes or slightly more, the film is removed from the holder and developed, whereby the fragment having a complementary sequence to the probe can be detected as a white band.

Using this technique of chemiluminescent labeling and photography in place of the troublesome radio-isotope-labeling method, which requires too long a time to obtain the result, the detection of a micro amount of a substance can be safely carried out in a short period of time.

The detecting sensitivity of the method of the present invention was determined in the following simple systems:

For 0-phenathroline derivatives, the detection of o-phenathroline of a concentration of up to $2.5 \times 10^{-5}$ M was possible by photographic chemiluminescence analysis, in a solution of a total amount of 35 ul containing $1.0 \times 10^{-2}$ M CEDAB (cationic surfactant), $3.5 \times 10^{-2}$ M sodium hydroxide, $4.3 \times 10^{-4}$ M copper (II) and 3.0 % hydrogen peroxide. When $6.7 \times 10^{-8}$ M osmium (VIII) was used in place of copper (II), the same detectable sensitivity was attained. This corresponds to $1.0 \times 10^{-9}$ mol of o-phenanthroline.

For luminol derivatives, detection was possible with $1.0 \times 10^{-7}$ M luminol, in a solution of a total amount of 30 $\mu$l containing $5.0 \times 10^{-8}$ M iron (III)-$\alpha,\beta,\gamma,$ $\delta$-tetrakis(4-carboxyphenyl)porphyrin (hereinafter referred to as TCPP) and 0.03 % hydrogen peroxide. This corresponds to $3.0 \times 10^{-12}$ mol of luminol. A specific sequence of 10 ug of PBR 322 vector can, for example, be detected within this detectable limit.

When a substrate is labeled with iron (III)-TCPP, detection of iron (III)-TCPP of a concentration of up to $6.7 \times 10^{-4}$ M was possible, in a solution of a total amount of 30 ul containing $1.0 \times 10^{-5}$ M luminol and 0.03 % hydrogen peroxide. This corresponds to $2.0 \times 10^{-18}$ mol of the catalyst iron (III)-TCPP. In plaque hybridization, $1 \times 10^{7}$ phages exist on average in one plaque, which corresponds to an amount of about $1.7 \times 10^{-17}$

mol. Accordingly, it is noted that the plaque may be sufficiently detected by the use of the method of the present invention.

For the system wherein the substrate is labeled with an acridinium compound, the detectable limit is up to about $8.0 \times 10^{-19}$ mol. Accordingly, the sensitivity in this system is even far higher than the aforesaid systems, and detection of a nucleic acid or protein of an ultra-micro amount is possible by the use of the system of using an acridinium compound.

While preferred methods of the present invention have been described above, the present invention is not limited to these embodiments. The method of the present invention may be applied to any and every labelable substrate and catalyst system. Such systems include, for example, oxalate derivatives such as bis(2,4,6-trichlorophenyl) oxalate; bis(2,4-dinitrophenyl) oxalate; bis(2,6-difluorophenyl) oxalate; and bis(pentafluorophenyl) oxalate, and enzymes such as peroxidase. Photographic films which may be used are not limited to the aforesaid Polaroid Type 612 Film or high-sensitive X-ray film, but any other photographic materials, including photographic films or photographic printing papers which have a sensitivity applicable to the object of the present invention, can of course be used.

The following non-limitative examples more particularly ilulstrate the present inveniton.

Example 1:

Method of photographically detecting a DNA fragment with a complementary sequence, using a luminol derivative-labeled DNA oligomer as a probe;

(1) Formation of DNA Oligomer and Probe:

Deoxy-oligonucleotides $5'T_{15} A3'$ (oligo dT) and $5'TA_{15}3'$ (oligo dA) were synsezied in a DNA synthesizer (380A, manufactured by Applied Biosystem Inc.). Oligo dT and ABEI were bonded to each other with glutaraldehyde via the amino group of the $3'$-terminal adenine. The reaction product was subjected to HPLC (ODS column; flow speed 1 ml/min; 20 minute-gradation of 10% acetonitrile/90% 10mM boric acid-triethylamine buffer - 50% acetonitrile/50% 10mM boric acid-triethylamine buffer) to thereby isolate and purify the ABEI-labeled compound of oligo dT (probe).

(2) Dot-blotting on Nitrocellulose Filter;

Oligo dA and oligo dT, each adjusted to have a concentration of $OD_{260}$-0.1, were spotted on a nitrocellulose filter (2 cm x 5 cm) each in an amount of 5 $\mu$l, 10 $\mu$l or 20 $\mu$l. The size of the dot was approximately 2 mm in diamter. In accordance with a conventional method (T. Maniatis et al, Molecular Cloning A Laboratory Manual, Cold Spring Harbor Laboratory, 1982, etc.), the filter was baked, put in a heat-sealed bag, prehybridized at 42°C and then hybridized, whereupon 100 $\mu$l of the probe prepared to have a concentration of $OD_{260}$ -1.0 was blended with 2 ml of the hybridization buffer. Ihe filter was then incubated at 42°C overnight, washed, dried with air, and then baked under reduced pressure.

(3) Detection of Chemiluminescence by Photography:

A rectangular container made of a thin plastic was set and fixed on a Polaroid Type 612 instant film cassette, the dimensions of the container corresponding substantially to that of the film cassette. The nitrocellulose filter was dipped in $3.0 \times 10^{-2}$M boric acid $6.0 \times 10^{-2}$ potassium hydroxide buffer and was placed inside the container and tightly attached thereto. The shutter cover of the film cassette was opened in a dark room, and 20 ml of aqueous hydrogen peroxide/iron(III)-TCPP solution (0.03 % hydrogen peroxide, $5.0 \times 10^{-8}$M iron (III)-TCPP/$3.0 \times 10^{-2}$M boric acid/$6.0 \times 10^{-2}$M potassium hydroxide buffer) was then poured into the container and allowed to stand as such for 10 minutes. The film was then removed from the cassette and developed, and the chemiliminescence in the film was ascertained. As a result, apparent white spots were found on 10 $\mu$l and 20$\mu$l of oligo dA among the dots applied, while no spot was found on oligo dT. It was thus established that the luminol-labeled oligo dT selectively hybridized with oligo dA having the complementary sequence thereto. The detectable sensitivity in the system was $6.0 \times 10^{-12}$ mol.

Example 2:

Method of photographically detecting a DNA fragment with a complementary sequence, using an iron(III)-TCPP catalyst-labeled DNA oligomer as a probe;

(1) Formation of Probe:

Iron(III)-TCPP was bonded to oligo dT as prepared in Example 1, with DCC (dicyclohexylcarbodiimide). The resulting iron(III)-TCPP-labeled oligo dT was isolated and purified by HPLC in the same manner as in Example 1.

(2) Southern Blotting on Nitrocellulose Filter:

Oligo dA was inserted into pBR322 at its EcoRI site using a conventional means for preparing a plasmid, and was then propagated in E. Coli 803. 2 μg of the resulting plasmid was cleaved with PstI and BamHI and subjected to agarose gel electrophoresis by setting on 0.8 % agarose gel in an amount of 0.1 μg, 0.5 μg and 1.0 μg, individually. At the same time, λ phage DNA was cleaved with HindIII, and was used as a marker. After electrophoresis, the agarose gel was dipped in sodium hydroxide/NaCl to produce a single-stranded DNA which was then transferred to a nitrocellulose filter. After being air-dried and then baked, the filter was put in a heat-sealed bag and thereafter subjected to pre-hybridization at 42° C, followed by hybridization, whereupon 100 μl of the probe prepared was blended with 3 ml of the hybridization buffer to have a concentration of $OD_{260} = 1.0$. Following incubation overnight at 42° C, the filter was washed, dried with air and then baked under reduced pressure.

(3) Detection of Chemiluminescence by Photography:

Using hydrogen peroxide and an aqueous isoluminol solution (0.3 % hydrogen peroxide, 1.0 x $10^{-5}$M isoluminol/3.0 x $10^{-2}$M boric acid 6.0 x $10^{-2}$M potassium hydroxide buffer), chemiluminescence was detected as in Example I. As a result, 1200 base-oligo dA-containing band only was detected in every sample of being 0.1μg, 0.5 μg or 1.0 μg. Accordingly, it was found that detection of even a trace amount of 3.4 x $10^{-14}$ mol or less is possible by the use of the system of Example 2. In detection of a specified base by Southern method, 0.1 to 1.0 μg of DNA is generally necessary. Accordingly, it is noted that the luminescent labeling of the present case is sufficiently substitutable for the conventional radioisotope-labeling.

Example 3:

Method of detecting a DNA to code the intended protein, using an acridinium derivative-labeled monoclonal antibody as a probe:

(1) Formation of Probe:

A monoclonal antibody capable of recognizing the carboxyl group site of proteoglycan of rat cancer cell strain L2 as an epitope was reacted with 4-(2-succinimidyloxycarbonylethyl)-phenyl-10-methylacridinium-9-carboxylate fluorosulfonate to give a probe.

(2) Screeing of DNA Library by Plaque Hybridization:

Using a DNA library of rat RBL1 cell strain as prepared with λ gt11, E. coli Y1090 was infected by a conventional method. Plaques were formed with 20 plates and then transferred to nitrocellulose filters, whereupon the number of the plaques was adjusted to be 1.0 x $10^4$/plate. The filter was set on a isoproply-β-D-galactose-containing agar medium and incubated overnight. The filter was then dipped in a solution comprising 10 ml of PBS buffer (pH 7.4) and an acridinium-labeled monoclonal antibody as blended with the buffer, for one hour, and then washed and dried with air.

(3) Detection of Chemiluminescence by Photography:

Using an aqueous hydrogen peroxide solution (0.3 % hydrogen peroxide, 0.1 sodium hydroxide, chemiluminescence was detected as in Example 1. Two spots were detected in 20 filters. These spots were found to be included in those as detected by screening with an L2 proteoglycan core protein-coding DNA as a probe.

Example 4:

Method of photographically detecting a DNA fragment with a complementary sequence, using a probe formed by labeling DNA with an iron(III)-TCPP catalyst via a spacer:

(1) Formation of Probe:

An iron(III)-TCPP was reacted with ethylenediamine with WSC and then was bonded to a 6kb DNA fragment (prepared by cleaving phage DNA with HindIII and isolating the cleaved fragment) via glutaraldehyde. The reaction mixture was purified by ethanol precipitation and ethanol washing.

(2) phage DNA was cleaved with HindIII and then subjected to agarose gel electrophoresis by setting on 0.8% agarose gel in an amount of 0.1 $\mu$g, 0.5 $\mu$g, and 1.0 $\mu$g, individually. Afterwards, the agarose gel was dipped in sodium hydroxide/NaCl to give a single-stranded DNA, which was then transferred to a nitrocellulose filter. The filter was, after dried with air and then baked under reduced pressure, put in a heat-sealed bag and thereafter subjected to pre-hybridization at 42°C followed by hybridization, whereupon 200 $\mu$l of the probe prepared was blended with the hybridization buffer to have a concentration of $OD_{260} = 1.0$. After incubated at 42°C overnight, the filter was washed, air-dried and then baked under reduced pressure.

(3) Detection of Chemiluminescence by Photography:

Using hydrogen peroxide and an aqueous luminol solution (0.1 % hydrogen peroxide, 3.4 x $10^{-2}$ M luminol/3.0 x $10^{-2}$M boric acid-6.0 x $10^{-2}$ potassium hydroxide buffer), chemiluminescence was detected as in Example 1. As a result, 6 kb band only was detected in every sample of being 0.1 $\mu$g, 0.5 $\mu$g or 1.0 $\mu$g.

**Claims**

1. A method of microanalysis, which comprises labeling a substrate containing material to be detected with a chemiluminescent substance, inducing a chemiluminescent reaction at the site of the labeled substrate, and photographically recording the reaction, whereby the site of the chemiluminescent reaction on the labeled substrate is detected and illustrated in a two-dimensional perspective.

2. A method according to claim 1, wherein the substrate is a nucleic acid or protein.

3. A method according to claim 1, which comprises labeling the substrate with an o-phenanthroline derivative, a luminol derivative, an acridinium derivative, an oxalate derivative, a metal ion, a metal ion chelate, or an enzyme.

4. A method according to claim 1, which comprises contacting the chemiluminescent substance with hydrogen peroxide, optionally in the presence of a suitable catalyst, to induce the chemiluminescent reaction.

5. A method according to claim 4, wherein the catalyst is copper(II) ion, osmium(VIII) ion, or iron-(III) ion.

6. A method according to claim 1, which comprises inducing the chemiluminescent reaction in close proximity to a highly sensitive instant photographic film.

7. A method according to claim 1, which comprises inducing the chemiluminescent reaction in close proximity to a highly sensitive X-ray film.

8. A method according to claim 1, which comprises photographing the chemiluminescent reaction with a camera having high sensitive film.

9. A method according to claim 3, which comprises labeling the substrate with an o-phenanthroline derivative.

10. A method according to claim 3, which comprises labeling the substrate with a luminol derivative.

11. A method according to claim 3, which comprises labeling the substrate with an acridinium derivative.

12. A method according to claim 3, which comprises labeling the substrate with an oxalate derivative.

13. A method according to claim 3, which comprises labeling the substrate with a copper (II) ion, osmium (VIII) ion, iron (III) ion, or a chelate thereof.

14. A method according to claim 10, wherein the luminol derivative is 6-N-(4-aminobutyl)-ethyl-2,3-dihydro-1,4-pthalhydrazinedione.

15. A method according to claim 12, wherein the oxalate derivative is bis (2,4-6-trichlorophenyl) oxalate, bis(2,4-dinitrophenyl) oxalate, bis (2,6-difluorophenyl) oxalate, or bis (pentafluorophenyl) oxalate.

16. A method according to claim 13, wherein the compound is iron (III)-$\alpha,\beta,\gamma;\delta$ -tetrakis(4-carboxyphenyl)porphyrin or a derivative thereof.

17. A method of microanalysis, which comprises

(a) labeling a substrate with a chemiluminescent substance;

(b) placing the labeled substrate on a suitable developing medium;

(c) placing the developing medium with the labeled substrate thereon in close proximity to highly sensitive instant photographic film or highly sensitive X-ray film;

(d) inducing a chemiluminescent reaction at the labeled site on the substrate; and

(e) exposing the film.

18. A method according to claim 17, wherein the the substrate is a nucleic acid or protein.

19. A method according to claim 17, which comprises labeling the substrate with an o-phenanthroline derivative, a luminol derivative, an acridinium derivative, an oxalate derivative, a metal ion, a metal ion chelate, or an enzyme.

20. A method according to claim 17, which comprises contacting the chemiluminescent substance with hydrogen peroxide, optionally in the presence of a suitable catalyst, to induce the chemiluminescent reaction.

21. A method according to claim 20, wherein the catalyst is copper (II) ion, osmium (VIII) ion, or iron (III) ion.

22. A method according to claim 17, which comprises labeling the substrate with an o-phenanthroline derivative.

23. A method according to claim 17, which comprises labeling the substrate with a luminol derivative.

24. A method according to claim 17, which comprises labeling the substrate with an acridinium derivative.

25. A method according to claim 17, which comprises labeling the substrate with an oxalate derivative.

26. A method accrding to claim 17, which comprises labeling the substrate with a copper (II) ion, osmium (VIII) ion, iron (III) ion, or a chelate thereof.

27. A method according to claim 23 , wherein the luminol derivative is 6-N-(4-aminobutyl)-ethyl-2,3-dihydro-1,4-pthalhydrazinedione.

28. A method according to claim 25, wherein the oxalate derivative is bis (2,4-6-trichlorophenyl) oxalate, bis(2,4-dinitrophenyl) oxalate, bis (2,6-difluorophenyl) oxalate, or bis (pentafluorophenyl) oxalate.

29. A method according to claim 26, wherein the compound is iron (III)-$\alpha,\beta$, $\gamma,\delta$ -tetrakis(4-carboxyphenyl)porphyrin or a derivative thereof.

30. A method according to claim 17, wherein the developing medium is a gel plate or nitrocellulose filter.

31. A method according to claim 17, wherein the developing medium and film are separated by a glass or plastic sheet or container.

32. A method according to claim 1, wherein the labeled substrate is a nucleic acid, and wherein prior to inducing the chemiluminescent reaction, the labeled substrate is hybridized with a nucleic . acid having a complementary base sequence.

33. A method according to claim 17, wherein the labeled substrate is a nucleic acid or protein, and which further comprises hybridizing the labeled substrate with a nucleic acid or protein having a complementary base sequence prior to inducing the chemiluminescent reaction.